Europäisches Patentamt

European Patent Office

Office européen des brevets

(11) Publication number: 0 402 951 A2

## (12) EUROPEAN PATENT APPLICATION

(21) Application number: 90111367.0

(22) Date of filing: 15.06.90

(51) Int. Cl.⁵: A61M 16/00

(30) Priority: 16.06.89 JP 71136/89 U
23.05.90 JP 134935/90

(43) Date of publication of application:
19.12.90 Bulletin 90/51

(84) Designated Contracting States:
CH DE FR GB LI SE

(71) Applicant: Akashi, Manabu
Esupoa Kakozan 701, 22, Kikusaka-cho
2-chome, Chikusa-ku
Nagoya-shi, Aichi-ken 464(JP)

(72) Inventor: Akashi, Manabu
Esupoa Kakozan 701, 22, Kikusaka-cho,
2-chome
Chikusa-ku, Nagoya-shi, Aichi-ken, 464(JP)
Inventor: Sakanaka, Kiyohiko
Houmei Student Heights, 47-3, Nishibasama
Nagakute, Nagakute-cho,
Aichi-ken,480-11(JP)

(74) Representative: Blumbach Weser Bergen
Kramer Zwirner Hoffmann Patentanwälte
Radeckestrasse 43
D-8000 München 60(DE)

(54) Mechanical ventilator for patient.

(57) A mechanical ventilator has an airway circuit (D) which is inserted into a trachea (T) of a patient by way of his airway, an inspiratory circuit (P1) and an expiratory circuit which are branched from the airway circuit. A gas delivering means (3) is provided at the end of the inspiratory circuit, and a gas flow regulating means is provided in the inspiratory circuit for regulating the gas flow to be delivered from the gas delivering means to the patient. A flowmeter (1a) is provided in the airway circuit (D) at the entry of the respiratory system of the patient for directly and continuously measuring the respiratory flow of the patient and outputting respiratory flow signal to a computer (5). The computer controls the operation of the gas flow regulating means so as to regulate the gas flow to be delivered to the patient in accordance with the change in respiratory flow signal of the patient.

## BACKGROUND OF THE INVENTION

### Field of the Invention

The present invention relates to a mechanical ventilator for artificially assisting the spontaneous breathing of a patient with respiratory failure.

### Description of the Prior Art

A mechanical ventilator for artificially assisting the spontaneous breathing of a patient with respiratory failure is required to maintain an airway pressure of the patient nearly constant over his entire respiratory cycle in order to ease dyspnea of the patient.

Furthermore, for a patient having only a small reserve respiration force, it is required to elevate the airway pressure in the inspiratory phase in order to assist the spontaneous breathing of such a patient.

The conventional ventilator has detecting means for detecting inspiration and expiration of a patient from a change in pressure in an inspiratory circuit and an expiratory circuit of the ventilator, or a change in gas flow in the inspiratory circuit or the expiratory circuit.

When the detecting means of the conventional ventilator detects the inspiration, gas flow is delivered to maintain the constant airway pressure or the desired level of the airway pressure. When the detecting means detects the expiration, the delivery of the gas flow is ceased or decreased.

However, in this conventional mechanical ventilator, the regulation of the gas flow is delayed from the time the respiratory flow of the spontaneous breathing of the patient actually changes because this conventional mechanical ventilator does not use the real respiratory flow of the patient in order to regulate the gas flow

In the conventional mechanical ventilator, in the beginning of the inspiratory phase, the delivery of the gas flow is delayed so that the airway pressure undesirably drops. In the expiratory phase, the airway pressure is undesirably elevated because of the resistance to the expiratory flow in the expiratory circuit. This is likely to cause the patient to complain of dyspnea.

Therefore, it has been demanded to develop a mechanical ventilator which does not cause the decrease in airway pressure during inspiration, especially during initial inspiration, and the increase in airway pressure during expiration.

### SUMMARY OF THE INVENTION

It is an object of the present invention to provide a mechanical ventilator by which the change in airway pressure of a patient can be reduced over his entire respiratory cycle, and accordingly dyspnea of the patient can be eased.

It is another object of the present invention to provide a mechanical ventilator by which the airway pressure of the patient can be arbitrarily regulated so as to match with the respiration force of the patient.

The mechanical ventilator in accordance with the present invention has measuring means for directly and continuously measuring the respiratory flow of a patient over his entire respiratory cycle and outputting respiratory flow signal, gas delivering means for delivering gas to the patient, gas flow regulating means for regulating the gas flow to be delivered to the patient, and control means for controlling the operation of the regulating means in accordance with the change in respiratory flow signal of the patient which is outputted by the measuring means.

In the mechanical ventilator having the above-described constitution, by the provision of the measuring means for measuring the respiratory flow of the patient directly and continuously, the gas flow to be delivered to the patient can be accurately regulated without any serious time lag in accordance with the actual change in respiratory flow of the patient. More specifically, in the inspiratory phase, the gas flow equivalent to the inspiratory flow of the patient is added to a predetermined basal flow, and in the expiratory phase, the gas flow equivalent to the expiratory flow is subtracted from the basal flow, and over the entire respiratory cycle, the necessary gas flow can be delivered to the trachea of the patient without any serious time lag behind the spontaneous breathing of the patient. The mechanical ventilator of the present invention can prevent the drop of the airway pressure in the beginning of the inspiratory phase, and the elevation of the airway pressure due to the resistance to the expiratory flow in the expiratory circuit in the expiratory phase, so as to remarkably reduce the change in airway pressure over the entire respiratory cycle of the patient Accordingly, dyspnea of the patient can be eased.

Furthermore, by integrating the respiratory flow signals of the patient measured by the measuring means, the tidal volume and the minute volume can be obtained. Based on the obtained tidal volume and minute volume, feed back control for obtaining more proper tidal volume and minute volume can be performed

The mechanical ventilator of the present invention can be further provided with airway pressure regulating means for regulating the pressure of the airway of the patient leading to his lung based on the respiratory flow signal of the patient measured by the respiratory flow measuring means. ·

By providing both the gas flow regulating means and the airway pressure regulating means, the change in airway pressure of the patient can be reduced to nearly constant, or the airway pressure of the patient can be arbitrarily regulated to match the patient's respiratory flow signal, thereby further easing dyspnea of the patient, particularly the patient having a small reserve respiration force.

From the airway pressure and tidal volume which are obtained by the respiratory flow signal outputted by the measuring means, the imposed work of breathing can be calculated. By regulating the gas flow to be added to or subtracted from the predexermined basal flow, the imposed work of breathing can be arbitrarily set. Or by setting the imposed work of breathing at a desired level, the gas flow and/or the airway pressure can be automatically set.

## BRIEF DESCRIPTION OF THE DRAWINGS

Fig. 1 is a schematic view of a main portion of a first embodiment of a mechanical ventilator in accordance with the present invention;

Fig. 2 is a schematic view of a main portion of a second embodiment of a mechanical ventilator in accordance with the present invention;

Fig. 3 is a schematic view of a main portion of a third embodiment of a mechanical ventilator in accordance with the present invention; and

Fig 4 is a schematic view of a main portion of a fourth embodiment of a mechanical ventilator in accordance with the present invention.

## DETAILED DESCRIPTION OF THE EMBODIMENTS

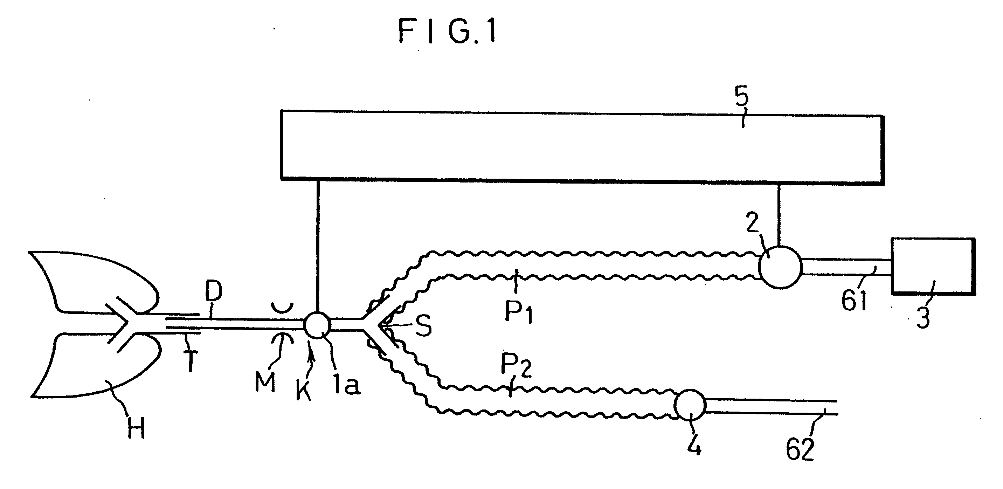

Fig. 1 illustrates a first embodiment of a mechanical ventilator in accordance with the present invention.

In Fig. 1, the mechanical ventilator has an endotracheal tube D which passes between lips M of a patient and is inserted into a trachea T of his lung H. tubes P1 and P2 which are connected to the endotracheal tube D through a branch connecting tube S. The endotracheal tube D can be inserted from the nostrils of the patient.

The endotracheal tube D serves as an airway circuit, the tube P1 serves as an inspiratory circuit, and the tube P2 serves as an expiratory circuit of the mechanical ventilator. Gas delivering means 3 is connected to an end of the tube P1 through a connecting tube 61 for delivering gas such as oxygen, air or the like into the tube P1. A constant-pressure valve 4 is provided at an end of the tube P2, and an open-ended connecting tube 62 is connected thereto.

Gas flow regulating means 2 for regulating the gas flow delivered by the gas delivering means 3 is provided in the tube P1 of the inspiratory circuit.

A flowmeter 1a for directly and continuously measuring the respiratory flow of the patient is provided between an end of the endotracheal tube D and an end of the branch connecting tube S at the entry K of the respiratory system of the patient. The flowmeter 1a is connected to control means 5 such as a computer, which is connected to the gas flow regulating means 2 for regulating the gas flow in the breathing circuit of the mechanical ventilator.

In the mechanical ventilator of the first embodiment having the above-described constitution, a predetermined basal flow is continuously delivered from the gas flow regulating means 2 into the tube P1 of the inspiratory circuit. The flowmeter 1a measures the respiratory flow of the patient, and inputs the respiratory flow signal to the controlling means 5, and the control means 5 controls the operation of the gas flow regulating means 2 in accordance with the respiratory flow signal. More specifically, during inspiration, a gas flow equivalent to the inspiratory flow of the spontaneous breathing of the patient is added to the basal flow, and during expiration, a gas flow equivalent to the expiratory flow of the spontaneous breathing of the patient is subtracted from the basal flow

In accordance with the mechanical ventilator of the first embodiment, by directly and continuously measuring the respiratory flow of the spontaneous breathing of the patient, the gas flow matching the patient's flow demand can be delivered to the patient continuously without any serious time lag. The change in airway pressure such as the drop thereof in the beginning of the inspiratory phase, and the elevation thereof in the expiratory phase, which are encountered in the conventional mechanical ventilator, can be markedly reduced, and the dyspnea of the patient can be greatly eased.

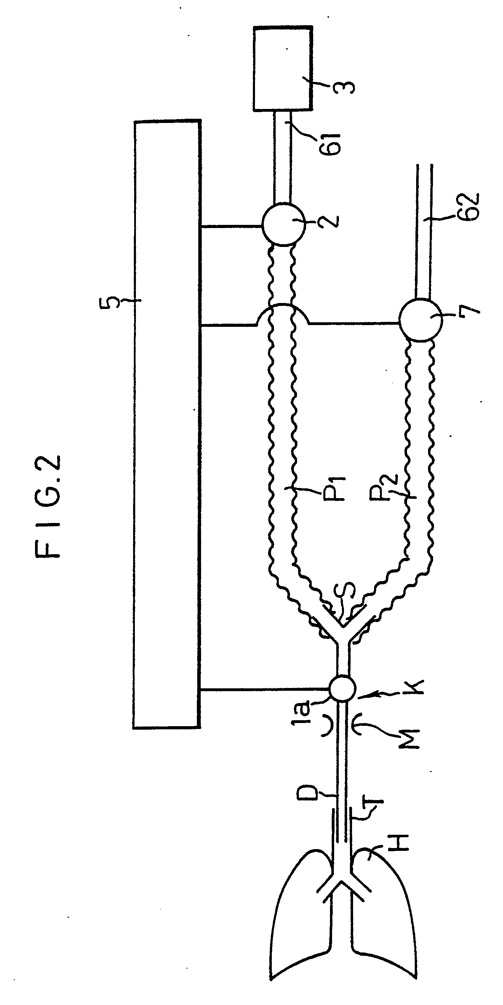

Fig. 2 illustrates a second embodiment of the present invention.

In Fig. 2, parts like or corresponding to those of Fig. 1 are designated by like reference characters. And the explanation of like parts will be omitted.

In the mechanical ventilator of the second embodiment, instead of the constant-pressure valve 4 of the first embodiment, an airway pressure regulating means 7 is provided at the end of the tube P2 of the expiratory circuit. The airway pressure regulating means 7 is connected to the control means 5.

In the mechanical ventilater of the second embodiment having the above-described constitution, the flowmeter 1a measures the respiratory flow of the spontaneous breathing of the patient and inputs respiratory flow signal to the control means 5, and the control means 5 controls the operation of the gas flow regulating means 2, resulting in the gas flow matching the patient's flow demand being delivered to the tube P1 of the inspiratory circuit over the entire respiratory cycle like the first embodiment.

Additionally, the control means 5 controls the operation of the airway pressure regulating means 7 in accordance with the respiratory flow signal measured by the flowmeter 1a, thereby regulating, if necessary, decreasing or increasing of the airway pressure.

The mechanical ventilator of the second embodiment can achieve the operational effect similar to that of the first embodiment. Furthermore, the airway pressure can be arbitrarily regulated so that the following additional operational effect can be achieved.

For a patient having only a small reserve respiration force, in the beginning of the inspiratory phase, the airway pressure is increased by decreasing the gas flow through the airway pressure regulating means 7 while in the expiratory phase, the airway pressure is decreased by increasing the gas flow through the airway pressure regulating means 7. The regulation of the airway pressure by the airway pressure regulating means 7 enables dyspnea of the patient having only a small reserve respiration force to be greatly eased.

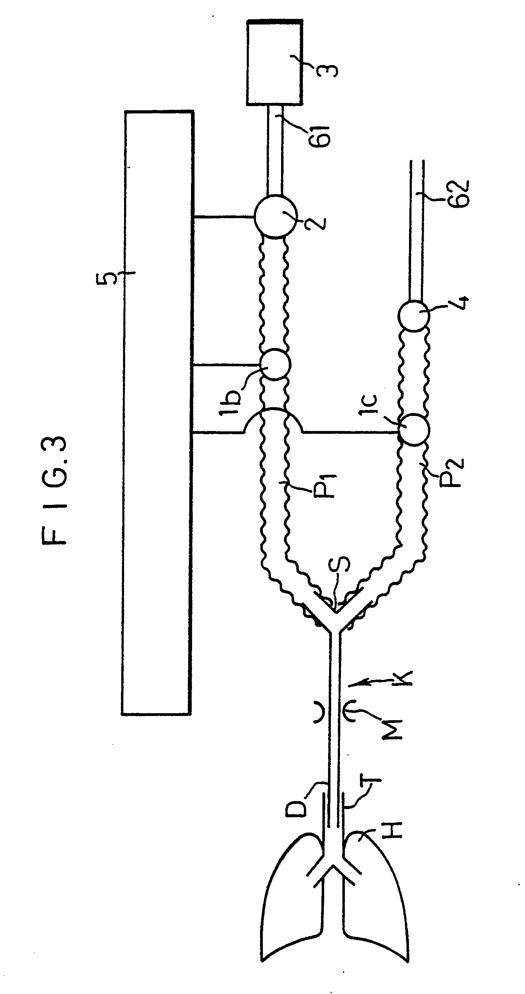

Fig. 3 illustrates a third embodiment of the present invention.

In Fig. 3, parts like or corresponding to those of Fig 1 are designated by like reference characters. Hereinafter, only the difference from the first embodiment will be explained.

In the first embodiment shown in Fig. 1, the respiratory flow of the patient is measured by the flowmeter 1a provided at the entry K of the respiratory system of the patient. In contrast, in the third embodiment, instead of the flowmeter 1a provided at the entry K of the respiratory system of the patient, flowmeters 1b, is provided in the tube P1 of the inspiratory circuit and another flowmeter 1c is provided in the tube P2 of the expiratory circuit. The flowmeters 1b, 1c are respectively connected to the control means 5, and respectively output gas flow signals. The mechanical ventilator of the third embodiment is further provided with operation means for calculating the respiratory flow of the patient based on the difference between the gas flow signals outputted by the flowmeters 1b and 1c. The operation means can be provided in each of the flowmeters 1b, 1c, within the control means 5, or lines between each flowmeter (1b, 1c) and the control means 5.

The respiratory flow signal calculated by the operation means based on the difference between the gas flow signals outputted by the flowmeters 1b, 1c is inputted to the control means 5, and based on the respiratory flow signal, the gas flow to be delivered to the patient is regulated by the regulating means 2 like the first embodiment.

The flowmeter 1b may be provided in the connecting tube 61 between the gas delivering means 3 and the gas flow regulating means 2, and the flowmeter 1c may be provided in the open-ended tube 62 extending outside of the airway pressure regulating means 7.

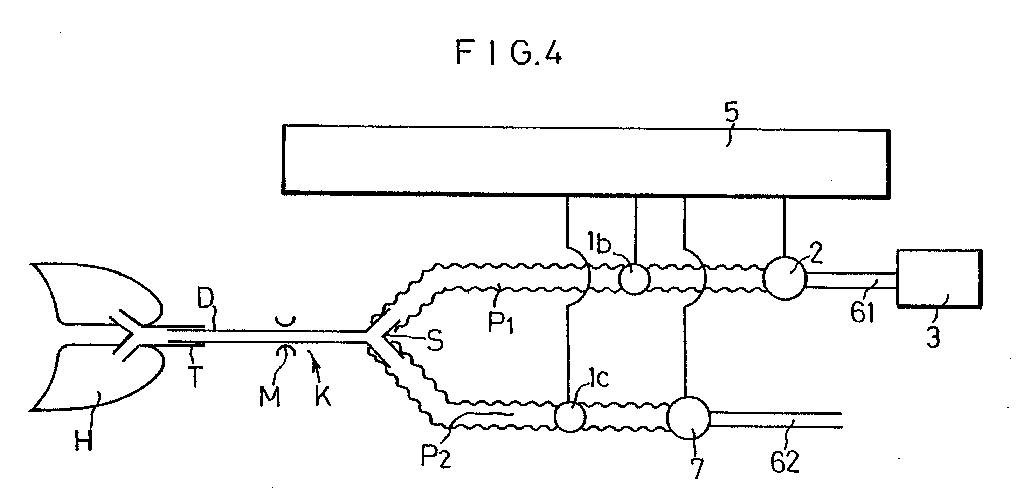

Fig 4 illustrates a fourth embodiment of the present invention. Parts like or corresponding to those of the second embodiment are designated by like reference characters. Hereinafter, only the difference from the second embodiment will be explained

In the second embodiment shown in Fig. 2, the respiratory flow of the patient is measured by the flowmeter 1a provided at the entry K of the respiratory system of the patient. In contrast, in the fourth embodiment, instead of the flowmeter 1a provided at the entry K of the respiratory system of the patient, flowmeters 1b, 1c are provided in the tube P1 of the inspiratory circuit and the tube P2 of the expiratory circuit like the third embodiment. The flowmeters 1b, 1c are respectively connected to the control means 5. The mechanical ventilator of the fourth embodiment is further provided with operation means for calculating the srespiratory flow signal of the patient based on the difference between gas flow signals outputted by the flowmeters 1b, 1c. The operation means can be provided in .each of the flowmeters 1b, 1c, within the control means 5, or in connecting lines between the flowmeters 1b, 1c and the control means 5.

In the fourth embodiment, the patient's respiratory flow signal is calculated by the operation means based on the difference between the gas flow signals outputted by the flowmeters 1b, 1c, and the gas flow to be delivered to the patient is regulated by the regulating means 2 based on the calculated respiratory flow signal like the third embodiment. Further, in the flourth embodiment, the airway pressure is regulated by the regulating means 7 based on the calculated respiratory flow signal like the second embodiment.

## Claims

1. A mechanical ventilator for a patient with respiratory failure, comprising:
respiratory flow measuring means (1a, 1b, 1c) for directly and continuously measuring a respiratory flow of the patient and outputting a respiratory flow signal;
gas delivering means (3) for delivering gas to the patient;
gas flow regulating means (2) for regulating the gas flow to be delivered to the patient; and
control means (5) for controlling the operation of said gas flow regulating means based on the respiratory flow signal outputted by said respiratory flow measuring means (1a, 1b, 1c).

2. A mechanical ventilator according to claim 1, further comprising:
airway pressure regulating means (4, 7) for regulating the pressure of an airway of the patient, leading to his lung;
said control means (5) further controlling the operation of said airway pressure regulating means (4, 7) based on the respiratory flow signal outputted by said respiratory flow measuring means (1a, 1b, 1c).

3. A mechanical ventilator according to claim 1, wherein said mechanical ventilator is provided with an airway circuit (D) leading to a trachea (T) of the patient by way of an airway of the patient, an inspiratory circuit (P1) and an expiratory circuit (P2) which are branched from said airway circuit (D), said gas delivering means (3) is provided so as to deliver gas to said inspiratory circuit (P1), and said gas flow regulating means (2) is provided in said inspiratory circuit.

4. A mechanical ventilator according to claim 2, wherein said mechanical ventilator is provided with an airway circuit (D) leading to a trachea (T) of the patient by way of an airway of the patient, an inspiratory circuit (P1) and an expiratory circuit (P2) which are branched from said airway circuit (D), said airway pressure regulating means (4, 7) is provided in said expiratory circuit (P2).

5. A mechanical ventilator according to claim 3, wherein said respiratory flow measuring means (1a) is provided in said airway circuit.

6. A mechanical ventilator according to claim 3, wherein said respiratory flow measuring means comprises gas flow measuring means (1b, 1c) provided in said inspiratory circuit (P1) and said expiratory circuit (P2) for measuring gas flow in said inspiratory circuit (P1) and said expiratory circuit (P2) and outputting gas flow signals, and operation means (2, 4, 7) for calculating the respiratory flow signal of the patient from the difference between the gas flow signals outputted by said gas flow measuring means, said operation means is provided in one of said gas flow measuring means, said control means, and connecting lines between said gas flow measuring means and said control means.

FIG.1

# FIG.2

EP 0 402 951 A2

# F I G.3

EP 0 402 951 A2

# F I G.4

EP 0 402 951 A2